# EUROPEAN PATENT APPLICATION

(11) **EP 4 138 518 A1**
(43) Date of publication of application: **22.02.2023**
(21) Application number: 21191613.5
(22) Date of filing: 17.08.2021
(51) Int. Cl.: H05B 47/16, H05B 47/115, A61N 5/06, A61M 21/00

(54) **CONTROL ENGINE FOR CONTROLLING A LIGHT ENVIRONMENT**

(71) Applicant: BrainLit AB, 223 63 Lund (SE)
(72) Inventor: Wingren, Tord, Lomma (SE); Lindoff, Bengt, Bjärred (SE); Singvall, Jacob, Bjärred (SE)
(74) Representative: AWA Sweden AB

(57) **Abstract**

A lighting system control engine for controlling a light environment at different locations is disclosed. The lighting system control engine comprising circuitry configured to: execute a light profile setting function configured to manipulate a template light profile describing illuminance and spectrum over a time period such that a first manipulated light profile associated with a first location and a second manipulated light profile associated with a second location are formed, said first and second manipulated light profiles are different, and said first and second locations are different; and execute a light source control function configured to, over time, control lighting at the first location in accordance with the first manipulated light profile and to, over time, control lighting the second location in accordance with the second manipulated light profile. Also, a lighting system including the control engine and a method for controlling light environments at different locations are disclosed.

## Description

### Technical field

The present disclosure relates to control of a light environment in a structure, such as a ship.

### Background of the invention

Chronobiology, which is a field of biology that studies timing (e.g., periodic) processes and phenomena in living organisms, is becoming increasingly more important for understanding human physiology. For example, the circadian rhythm is an internal process of living organisms (e.g. humans) that regulates the sleep-wake cycle. The circadian rhythm typically repeats over a time period which, for most people, is slightly longer than 24 hours. It is also known that this cycle can be different between different individuals. For example, some persons are known to be "morning" persons, while other are "evening" persons. Hence, the behavior at a certain time of day for different individual typically varies. This is usually referred to as a person's chronotype.

An important part of chronobiology is the study of the impact of light on living organisms. It has, for example, been found that exposing a person to bright light in the evening can postpone the time that person falls asleep. Recent studies have further found that light can affect a person's alertness and performance. However, the effect of light at a certain time of the day is not only individual and dependent on time of the day, it also dependent on the individual persons light exposure during the day. For instance, in case the person has been exposed to day light in the morning (e.g., light similar to that of a bright summer's morning), the effect of bright light in the afternoon is typically less than is the person had spent the morning being exposed to light of lower illuminance (e.g. artificial light inside an office).

Further, it is well known that shift workers have higher risk of medical conditions such as cancer and cardiovascular disease, as well as depression. This is mainly due to the disturbance of the circadian rhythm due to the need for the individual person to work during hours when the body is prepared for sleep. Hence, there is a need for improving the wellbeing of shift workers. This is especially important for shift workers onboard on e.g. ships or oil rigs where the workers are onboard 24 hours a day 7 days a week, and the ship or oil rig requires 24 hours a day crew. Another area of improving wellbeing due to the disturbance of the circadian rhythm is for the crew on long-haul flights.

### Summary of the invention

It is an object to, at least partly, mitigate, alleviate, or eliminate one or more of the above-identified deficiencies in the art and disadvantages singly or in any combination and solve at least the above-mentioned problem.

According to a first aspect lighting system control engine for controlling a light environment at different locations is provided. The lighting system control engine comprising circuitry configured to: execute a light profile setting function configured to manipulate a template light profile describing illuminance and spectrum over a time period such that a first manipulated light profile associated with a first location and a second manipulated light profile associated with a second location are formed, said first and second manipulated light profiles are different, and said first and second locations are different; and execute a light source control function configured to, over time, control lighting at the first location in accordance with the first manipulated light profile and to, over time, control lighting at the second location in accordance with the second manipulated light profile. The light profile setting function is configured to form the first manipulated light profile by: extracting a first sub portion of the template light profile corresponding to a first sub time period of the time period of the template light profile; and arranging multiple versions of the first sub portion of the template light profile timewise after each other. The multiple versions of the first sub portion of the template light profile may be arranged timewise directly after each other. The light profile setting function is configured to form the second manipulated light profile by: extracting a second sub portion of the template light profile corresponding to a second sub time period of the time period of the template light profile; and arranging multiple versions of the second sub portion of the template light profile timewise after each other. The multiple versions of the second sub portion does not need to be arranged timewise directly after each other. As will be discussed below other portions of the template light profile may be inserted in-between two of the versions of the second sub portion.

The present control engine provides for improved wellbeing of shift workers, e.g. onboard a ship. This since each shift of workers may live their life according to their circadian rhythm. Further, energy may be saved by optimizing the light profile for respective locations, to achieve optimal light for the different locations at each time.

The light profile setting function may further be configured to: extract a third sub portion of the template light profile corresponding to a third sub time period of the template light profile; and add multiple versions of the third sub portion of the template light profile to the second manipulated light profile.

The third and second sub portions of the template light profile may be interleaved in the second manipulated light profile.

The template light profile may be describing illuminance and spectrum for a day and night cycle.

The first sub time period of the time period of the template light profile may correspond to daytime working hours. The second sub time period of the time period of the template light profile may correspond to morning and/or evening hours.

According to a second aspect lighting system is provided. The lighting system comprising a light system control engine according the first aspect; a first light source configured to illuminate the first location; and a second light source, separate from the first light source, configured to illuminate the second location.

The first and second locations may be different rooms in a same structure. The same structure may be one of: a ship; an airplane; an oil rig; a space shuttle; a lunar base camp; a planet base camp; a cave; or a mine.

The first and second locations may be at different places. For example, the first location may be at an airplane and the second location may be at a hotel room or the like where a member of the airplane crew is spending time in-between two flights.

The above-mentioned features of the first aspect, when applicable, apply to this second aspect as well. In order to avoid undue repetition, reference is made to the above.

According to a third aspect a method for controlling light environments at a first and a second location is provided. The second location being different from the first location. The method comprising: manipulating a template light profile describing illuminance and spectrum over a time period such that a first manipulated light profile associated with the first location and a second manipulated light profile associated with the location are formed. Said first and second manipulated light profiles are different. The method further comprising controlling, over time, one or more light sources at the first location in accordance with the first manipulated light profile; and controlling, over time, one or more light sources at the second location in accordance with the second manipulated light profile. Forming the first manipulated light profile comprises: extracting a first sub portion of the template light profile corresponding to a first sub time period of the time period of the template light profile, and arranging multiple versions of the first sub portion of the template light profile timewise after each other. Forming the second manipulated light profile comprises: extracting a second sub portion of the template light profile corresponding to a second sub time period of the time period of the template light profile, the second sub portion being different from the first sub portion, and arranging multiple versions of the second sub portion of the template light profile timewise after each other.

Forming the second manipulated light profile may further comprise: extracting a third sub portion of the template light profile corresponding to a third sub time period of the template light profile; and adding multiple versions of the third sub portion of the template light profile to the second manipulated light profile. The third and second sub portions of the template light profile may be interleaved in the second manipulated light profile.

The above-mentioned features of the first and second aspects, when applicable, apply to this third aspect as well. In order to avoid undue repetition, reference is made to the above.

According to a fourth aspect a non-transitory computer-readable storage medium is provided. The non-transitory computer-readable storage medium having stored thereon instructions for implementing the method according to the third aspect, when executed on a device having processing capabilities.

A further scope of applicability will become apparent from the detailed description given below. However, it should be understood that the detailed description and specific examples are given by way of illustration only.

It is to be understood that the terminology used herein is for purpose of describing particular embodiments only, and is not intended to be limiting. It must be noted that, as used in the specification and the appended claim, the articles "a," "an," "the," and "said" are intended to mean that there are one or more of the elements unless the context clearly dictates otherwise. Thus, for example, reference to "a unit" or "the unit" may include several devices, and the like. Furthermore, the words "comprising", "including", "containing" and similar wordings does not exclude other elements or steps.

### Brief description of the drawings

The above and other aspects will now be described in more detail, with reference to appended figures. The figures should not be considered limiting; instead they are used for explaining and understanding.

As illustrated in the figures, the sizes of layers and regions may be exaggerated for illustrative purposes and, thus, are provided to illustrate the general structures. Like reference numerals refer to like elements throughout.
Fig. 1 illustrates a lighting system arranged in a structure 5.
Fig. 2 illustrates a control engine of the lighting system of Fig. 1.
Fig. 3 illustrates manipulation of a template light profile for forming manipulated light profiles.
Fig. 4 is a block diagram of a method for controlling a light environment at a first and a second location.

### Detailed description

The present invention will now be described more fully hereinafter with reference to the accompanying drawings, in which currently preferred embodiments of the invention are shown. This invention may, however, be embodied in many different forms and should not be construed as limited to the embodiments set forth herein; rather, these embodiments are provided for thoroughness and completeness, and to fully convey the scope of the invention to the skilled person.

The concept of the present invention is independently controlling light sources at a plurality of locations in for instance a ship, an airplane, an oil rig, a space shuttle, a lunar base camp, a planet base camp, a cave, or a mine. The light sources are controlled such that a light environment is set to be different at the different locations. Within the context of this disclosure, the wording "light environment" should be construed as spectrally and temporally resolved amount of light emitted from light source(s) at a location. The light source(s) is/are controlled using a light profile associated with the respective location. The location specific light profile comprises spectrally and temporally resolved information about amount of light to be emitted from light source(s) at a specific location. Since the information is spectrally and temporally resolved, the light profile comprise information on the amount of light at which time and at which wavelength/color the light source(s) at a specific location is/are set to emit. For instance, the light profile for light source(s) in a first location may follow a first sub portion of a template light profile repetitively and the light profile for light source(s) in a second location may follow a second sub portion of the template light profile repetitively. The first location (e.g. an engine room in a ship, a cock-pit in an airplane, etc.) may be set to repetitively follow a "mid-day" sub-portion of the template light profile and the second location (e.g. a "spare time" room in the ship, a bed room of an individual, etc.) may be set to repetitively follow a "morning+evening" sub-portion of the template light profile. Hence, the template light profile may describe illuminance and spectrum for a day and night cycle (24 hours), especially illuminance and spectrum in an outdoor day and night cycle.

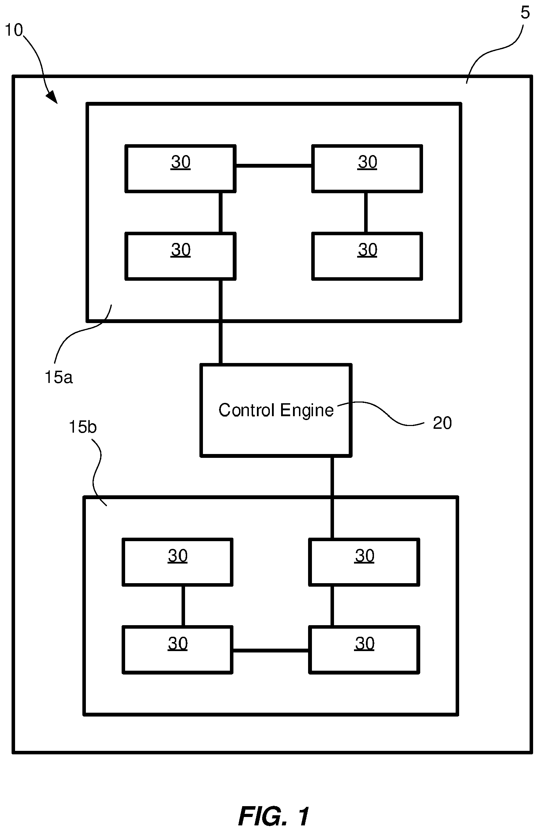

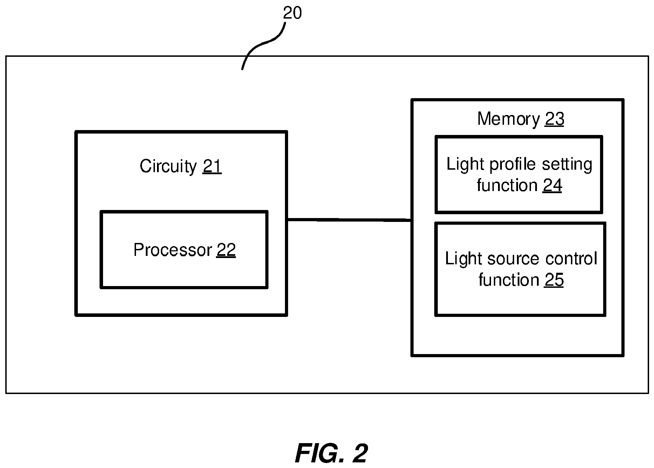

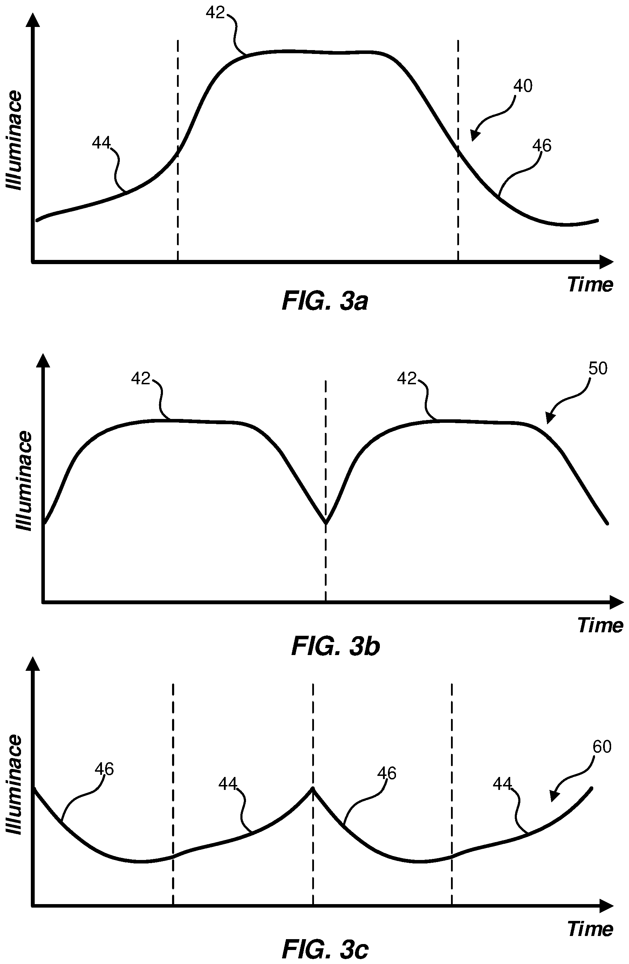

In connection with Figs 1-3 the above discussed lighting system and in particular a control engine for such a lighting system will be discussed. Fig. 1 illustrates a lighting system 10 arranged in a structure 5. The structure 5 may e.g. be a ship. Fig. 2 illustrates a control engine 20 of the lighting system 10. Fig. 3 illustrates manipulation of the template light profile for forming manipulated light profiles.

Fig. 1 illustrates a lighting system 10 arranged in a structure 5, e.g. a ship. The structure 5 comprises a plurality of locations 15a, 15b. Each location 15a, 15b may be a separate room or separate area in the structure 5. The lighting system 10 comprises a plurality of light sources 30. A subset of the plurality of light sources 30 is located in each location 15a, 15b. Each subset of the plurality of light sources 30 may be one or more light sources 30. In case a subset of the plurality of light sources 30 is two or more light sources 30, the light sources 30 may be of a same or different type. The light sources 30 of the same or different type may be controlled individually or jointly. The lighting system 10 further comprises a control engine 20. The control engine 20 is configured to control illumination of the plurality of light sources 30. More specifically, the control engine 30 is configured to control illumination of each of the subsets of light sources 30 individually. Hence, the control engine 30 is configured to control illumination in each location 15a, 15b individually. As will be discussed in greater detail below, the control engine 30 is configured to base the controlling at the different locations 15a, 15b on a light profile dedicated for the specific location 15a, 15b.

According to a non-limiting example, the lighting system 10 is arranged at a ship. Such a ship may be out on the ocean for weeks or months. Typically, 24 hours operation is needed where the crew onboard the ship is working in shifts, for instance 12 hours work, and 12 hours off-duty. Each shift may comprise of one or more individuals in the crew. Work location(s) are typically separated from off-duty location(s). In order to achieve optimal work performance for the crew, the respective shift should follow their circadian rhythm. That is, each shift shall have their "mid-day" light at work location(s), while they should have morning and/or evening light in off-duty location(s). A work location may be seen as an example of a first location 15a serviced by the lighting system 10. An off-duty location may be seen as a second location 15b serviced by the lighting system 10. For example, the first location 15a may be an engine room of the ship, whereas the second location 15b may be a room for leisure time onboard the ship. In the first location 15a "mid-day" light conditions should be mimicked for every shift and in the second location 15b "morning+evening" light should be mimicked for every shift. Hence, the control engine 20 should be configured to control the light at the different locations 15a and 15b differently. The controlling of the light at the different locations 15a, 15b is based on a light profile dedicated for the specific location.

In connection with Fig. 2, the control engine 20 will be discussed in more detail. The control engine may be embodied in a specific device, e.g. a server. However, as readily understood by the skilled person the control engine 10 may be distributed over a plurality of devices. The control engine 20 comprises circuitry 21. The circuitry 21 may include a processor 22, such as a central processing unit (CPU), microcontroller, or microprocessor. The circuitry 21 is configured to execute program code stored in a memory 23, in order to carry out functions and operations of the control engine 20.

The memory 23 may be one or more of a buffer, a flash memory, a hard drive, a removable medium, a volatile memory, a non-volatile memory, a random access memory (RAM), or another suitable device. In a typical arrangement, the memory 23 may include a non-volatile memory for long term data storage and a volatile memory that functions as system memory for the control engine 20. The memory 23 may exchange data with the circuitry 21 over a data bus. Accompanying control lines and an address bus between the memory 23 and the circuitry 21 also may be present.

Functions and operations of the control engine 20 may be embodied in the form of executable logic routines (e.g., lines of code, software programs, etc.) that are stored on a non-transitory computer readable medium (e.g., the memory 23) of the control engine 20 and are executed by the processor 21. Furthermore, the functions and operations of the control engine 20 may be a stand-alone software application or form a part of a software application that carries out additional tasks related to the control engine 20. The described functions and operations may be considered as part of a method that the control engine 20 is configured to carry out. Also, while the described functions and operations may be implemented in software, such functionality may as well be carried out via dedicated hardware or firmware, or some combination of hardware, firmware and/or software.

The control engine 20 is configured to execute a light profile setting function 24. The light profile setting function 24 is configured to manipulate a template light profile 40. An example of a template light profile 40 is illustrated in Fig. 3a. The template light profile 40 is describing illuminance and spectrum over a time period. Typically, the template light profile 40 is describing illuminance and spectrum for a day and night cycle (25 hours), especially for an outdoor day and night cycle. The in Fig. 3a illustrated template light profile 40 is just a part of the template light profile 40 for one specific wavelength of light. The illuminance for different wavelengths of the light spectrum will vary in a similar manner over time. However, as readily understood by the skilled person e.g. red and blue light may vary differently throughout a day and night cycle.

The light profile setting function 24 is configured to manipulate the template light profile 40 by portioning the template light profile 40 into different sub portions 42, 44, 46. Hence, light profile setting function 24 is configured to extract sub portions 42, 44, 46 from the template light profile 40. A first sub portion 42 of the template light profile 40 correspond to first sub time period. The first sub time period may e.g. correspond to "mid-day" or daytime working hours. For a two-shift set-up the first sub time period may correspond to 06.00 to 18.00, this is the case illustrated in Fig. 3a. For a three-shift set-up the first sub time period may correspond to 08.00 to 16.00. A second sub portion 44 of the template light profile 40 correspond to second sub time period. The second sub time period may e.g. correspond to "morning" hours. For a two-shift set-up the second sub time period may correspond to 00.00 to 06.00, this is the case illustrated in Fig. 3a. For a three-shift set-up the second sub time period may correspond to 04.00 to 08.00. A third sub portion 46 of the template light profile 40 correspond to third sub time period. The third sub time period may e.g. correspond to "evening" hours. For a two-shift set-up the third sub time period may correspond to 18.00 to 24.00, this is the case illustrated in Fig. 3a. For a three-shift set-up the second sub time period may correspond to 16.00 to 20.00. Hence, timewise the order of the first, second and third sub portions 42, 44, 46 of the template light profile 40 are arranged in the following order: the second sub portion 44 followed by the first sub portion 42 and then followed by the third sub portion 46.

The light profile setting function 24 is configured to form specific manipulated light profiles for the different locations 15a, 15b serviced by the lighting system 10. The so formed specific manipulated light profiles for the different locations 15a, 15b are typically different. For example, the light profile setting function 24 is configured to form a first manipulated light profile 50 associated with the first location 15a and a second manipulated light profile 60 associated with the second location 15b. Examples of first and second manipulated light profiles 50, 60 are illustrated in Figs 3b and 3c, respectively, which now will be discussed in more detail.

The first manipulated light profile 50 may be formed by extracting the first sub portion 42 of the template light profile 40 and arranging multiple versions of the first sub portion 42 of the template light profile 40 timewise after each other, preferably directly after each other. The in Fig. 3a illustrated example of a first manipulated light profile 50 is for a two-shift set-up. It is however, readily understood that a first manipulated light profile 50 for a three-shift or higher number of shifts may also be formed. The first manipulated light profile 50 should typically mimic daytime working hours. Hence, the first manipulate light profile 50 associated with the first location 15a follows a midpart of the template light profile 40. For a two-shift set-up the first manipulate light profile 50 follows the midpart of the template light profile 40 twice per 24 hours, i.e. the first sub portion 42 of the template light profile 40 corresponds to 12 hours, this is illustrated in Fig. 3b. For a three-shift set-up the first manipulate light profile 50 should in instead follow the midpart of the template light profile 40 three time per 24 hours, i.e. the first sub portion 42 of the template light profile 40 corresponds to 8 hours. When a shift is leaving the first location 15a (for possibly going to the second location 15b) a new start on the working day for the new shift entering the first location 15a is starting at the first location 15a.

The second manipulated light profile 60 may be formed by extracting the second and third sub portions 44, 46 of the template light profile 40 and arranging multiple versions of the second and third sub portions 44, 46 of the template light profile 40 timewise after each other. The in Fig. 3c illustrated example of a second manipulated light profile 60 is for a two-shift set-up. It is however, readily understood that a second manipulated light profile 60 for a three-shift or higher number of shifts may also be formed. The second manipulated light profile 60 should typically mimic morning and evening hours. Hence, the second manipulate light profile 60 associated with the second location 15b follows morning and evening portions of the template light profile 40. For a two-shift set-up the second manipulate light profile 60 follows the morning and evening of the template light profile 40 twice per 24 hours, i.e. the second sub portion 44 of the template light profile 40 corresponds to 6 hours, and the third sub portion 46 of the template light profile 40 also corresponds to 6 hours, this is illustrated in Fig. 3c. For a three-shift set-up the second manipulate light profile 60 should in instead follow the second and third portions 44, 46 of the template light profile 40 three time per 24 hours, i.e. both the second and third sub portions 44, 46 of the template light profile 40 corresponds to 4 hours each. Accordingly, the second manipulate light profile 60 should mimic off-duty hours. When a shift is leaving the second location 15b (for possibly going to the first location 15a) a new start of an "evening" for the shift leaving the first location 15a is starting at the second location 15b.

Preferably, the second manipulated light profile 60 is synchronized with the first manipulated light profile 50. Typically, the second manipulated light profile 60 is synchronized with the first manipulated light profile 50 so that when a first sub portion 42 of the first manipulated light profile 50 is ending a third sub portion 46 of the second manipulated light profile 60 is beginning. Further, typically, the second manipulated light profile 60 is synchronized with the first manipulated light profile 50 so that when a first sub portion 42 of the first manipulated light profile 50 is beginning a first sub portion 44 of the second manipulated light profile 60 is ending.

The control engine 20 is further configured to execute a light source control function 25. The light source control function 25 is configured to control the plurality of light sources 30 of the lighting system 10. The light source control function 25 is configured to, over time, control the light sources at the first location 15a in accordance with the first manipulated light profile 50. Further, the light source control function 25 is configured to, over time, control the light sources at the second location 15b in accordance with the second manipulated light profile 60. In this manner it may be safeguarded that an optimal lighting environment will be set at the different locations 15a, 15b of the structure 5 serviced by the lighting system 10.

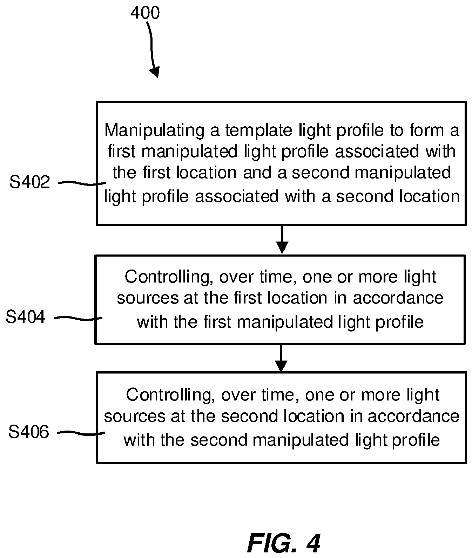

In connection with Fig. 4 a method 400 for controlling light environments at the first and the second locations 15a, 15b will be discussed. Some of all the steps of the method 400 may be performed by the functions of the control engine 20 described above. However, it is equally realized that some or all of the steps of the method 400 may be performed by similar functions performed at other devices. The method 400 comprises the following steps. The steps may be performed in any suitable order. The method comprises the following steps.

Manipulating S402 the template light profile 40. The template light profile 40 describing illuminance and spectrum over a time period. The manipulating S402 being performed such that a first manipulated light profile 50 associated with the first location 15a and a second manipulated light profile 60 associated with the second location15b are formed. The formation of the manipulated light profiles is discussed in more detail above in connection with Fig. 3 and in connection with the light profile setting function 24. In order to avoid undue repetition reference is made to the above.

Controlling S404, over time, one or more light sources at the first location 15a in accordance with the first manipulated light profile 50.

Controlling S406, over time, one or more light sources at the second location 15b in accordance with the second manipulated light profile 60.

The person skilled in the art realizes that the present invention by no means is limited to what is explicitly described above. On the contrary, many modifications and variations are possible within the scope of the appended claims.

For example, the template light profile 40 may be an individual template light profile. That is, the template light profile 40 may be a light profile optimized for a specific individual. If so, the first and/or second manipulated light profiles 50, 60 may also be individual manipulated light profiles. This may be implemented in case the first and/or second location 15a, 15b is occupied by only one individual. For example, in case the second location 15b is a location, e.g. a personal bedroom, of a specific individual, the second manipulated light profile 60 may be based on a template light profile 40 for that specific individual. By in this way base the first and/or second manipulated light profiles 50, 60 on an individual template light profile, optimized well being for the specific individual may be achieved. In connection with this, the lighting system 10 may further comprise a of an individual user detector. The individual user detector being configured to detect which individual user that is present at the first and/or second location 15a, 15b. Hence, a specific individual user detector may be arranged at each of the first and the second location 15a, 15b. The individual user detector may e.g. be embodied as a camera e.g. running a face recognition algorithm. Alternatively, or in combination, the individual user detector may e.g. be embodied as a device configured to detect presence of a smart device (e.g. smart phone, smart watch) associated with a specific individual. Alternatively, or in combination, data from an access granting system or the like may be used to detect which individual user that is present at the first and/or second location 15a, 15b. As readily understood by the skilled person, the individual user detector may be embodied in other ways as long as the device is configured to detect which individual user that is present at a specific location.

Alternatively, or in combination, the template light profile 40 may be a common template light profile. If so, the template light profile 40 may be a general light profile following a typical day and night cycle. Alternatively, the common template light profile may be a template light profile based on an average of different individual template light profiles, e.g. for the workers of the specific shift. The common template light profile may be determined based on data from an individual user detector (as discussed above) arranged at the location whereat the light environment is controlled. That is, after having determined the different individuals present at a location the template light profile may be determined and thereafter the manipulated light profile for that location may be formed.

Moreover, for locations not being dedicated for a specific shift, e.g. rest rooms, hallways etc., a neutral light profile may be used to form the light environment. Alternatively, at such locations there may be installed individual user detectors (as discussed above) and the light environment may be adopted based on individual(s) presently occupying the location.

Additionally, variations can be understood and effected by the skilled person in practicing the claimed invention, from a study of the drawings, the disclosure, and the appended claims.

## Claims

1. A lighting system control engine for controlling a light environment at different locations, the lighting system control engine comprising circuitry configured to:
execute a light profile setting function configured to manipulate a template light profile describing illuminance and spectrum over a time period such that a first manipulated light profile associated with a first location and a second manipulated light profile associated with a second location are formed, said first and second manipulated light profiles are different, and said first and second locations are different,
wherein the light profile setting function is configured to form the first manipulated light profile by:
extracting a first sub portion of the template light profile corresponding to a first sub time period of the time period of the template light profile, and
arranging multiple versions of the first sub portion of the template light profile timewise after each other; and
wherein the light profile setting function is configured to form the second manipulated light profile by:
extracting a second sub portion of the template light profile corresponding to a second sub time period of the time period of the template light profile, and
arranging multiple versions of the second sub portion of
the template light profile timewise after each other; and
execute a light source control function configured to, over time, control lighting at the first location in accordance with the first manipulated light profile and to, over time, control lighting at the second location in accordance with the second manipulated light profile.

2. The light system control engine according to claim 1, wherein the light profile setting function is further configured to:
extract a third sub portion of the template light profile corresponding to a third sub time period of the template light profile; and
add multiple versions of the third sub portion of the template light profile to the second manipulated light profile.

3. The light system control engine according to claim 2, wherein the third and second sub portions of the template light profile are interleaved in the second manipulated light profile.

4. The light system control engine according to any one of claims 1-3, wherein the template light profile is describing illuminance and spectrum for a day and night cycle.

5. The light system control engine according to claim 4, wherein the first sub time period of the time period of the template light profile corresponds to daytime working hours.

6. The light system control engine according to claim 4 or 5, wherein the second sub time period of the time period of the template light profile corresponds to morning and/or evening hours.

7. A lighting system comprising:
a light system control engine according to any one of claims 1-6;
a first light source configured to illuminate the first location;
a second light source, separate from the first light source, configured to illuminate the second location.

8. The lighting system according to claim 7, wherein the first and second locations are different rooms in a same structure.

9. The lighting system according claim 8, wherein the same structure is one of:
a ship;
an airplane;
an oil rig;
a space shuttle;
a lunar base camp;
a planet base camp;
a cave; or
a mine.

10. A method for controlling light environments at a first and a second location, the second location being different from the first location, the method comprising:
manipulating a template light profile describing illuminance and spectrum over a time period such that a first manipulated light profile associated with the first location and a second manipulated light profile associated with the location are formed, said first and second manipulated light profiles are different,
wherein forming the first manipulated light profile comprises:
extracting a first sub portion of the template light profile corresponding to a first sub time period of the time period of the template light profile, and
arranging multiple versions of the first sub portion of the template light profile timewise after each other;
wherein forming the second manipulated light profile comprises:
extracting a second sub portion of the template light profile corresponding to a second sub time period of the time period of the template light profile, the second sub portion being different from the first sub portion, and
arranging multiple versions of the second sub portion of the template light profile timewise after each other;
controlling, over time, one or more light sources at the first location in accordance with the first manipulated light profile;
controlling, over time, one or more light sources at the second location in accordance with the second manipulated light profile.

11. The method according to claim 10, wherein forming the second manipulated light profile further comprises:
extracting a third sub portion of the template light profile corresponding to a third sub time period of the template light profile; and
adding multiple versions of the third sub portion of the template light profile to the second manipulated light profile.

12. The method according to claim 10 or 11, wherein the template light profile is describing illuminance and spectrum for a day and night cycle.

13. The method according to claim 12, wherein the first sub time period of the time period of the template light profile corresponds to daytime working hours.

14. The method according to claim 12 or 13, wherein the second sub time period of the time period of the template light profile corresponds to morning and/or evening hours.

15. A non-transitory computer-readable storage medium having stored thereon instructions for implementing the method according to any one of claims 10-14, when executed on a device having processing capabilities.
